# EUROPEAN PATENT SPECIFICATION

(11) **EP 1 253 849 B1**
(45) Date of publication and mention of the grant of the patent: **05.01.2005**
(21) Application number: 01908932.5
(22) Date of filing: 07.02.2001
(51) Int. Cl.: A61B 1/12

(54) **FLUID CONNECTION SYSTEM FOR ENDOSCOPE REPROCESSING WITH CONTROLLED LEAKAGE**
FLÜSSIGKEITSVERBINDUNGSVORRICHTUNG FÜR ENDOSKOP MIT KONTROLLIERBARER FLUIDLECKAGE
SYSTEME DE RACCORDEMENT FLUIDIQUE DESTINE AU RETRAITEMENT ENDOSCOPIQUE AVEC DISPOSITIF DE DETECTION DE FUITES

(30) Priority: 07.02.2000 US 499134
(43) Date of publication of application: 06.11.2002
(73) Proprietor: STERIS INC., Temecula, California 92590 (US)
(72) Inventor: MAPSON, Tara, Denise, Cleveland, OH 44110 (US); KESELMAN, Yury, Beachwood, OH 44122 (US); JESURUN, David, South Euclid, OH 44121 (US); SELIG, Victor, M., Euclid, OH 44123 (US); TVERGYAK, Joseph, Chardon, OH 44024 (US); SCHIEMAN, Richard, A., Novelty, OH 44072 (US); VAVRA, Bruce, L., Wickliffe, OH 44092 (US); SESTAK, Joseph, T., Erie, PA 16506 (US)
(74) Representative: Leineweber, Jürgen, Dipl.-Phys.
(86) International application number: PCT/US2001/003962
(87) International publication number: WO 2001/056459

(56) References cited:
- EP-A- 0 835 665
- EP-A- 0 945 140
- DE-A- 3 416 743
- DE-A- 3 538 744
- US-A- 5 505 218
- US-A- 6 041 794

## Description

### Background of the Invention

The present application relates to the fluid handling arts. It finds particular application in conjunction with fluid sterilization and disinfection systems and will be described with reference thereto.

Fluid sterilization and disinfection systems are typically designed to cause microbes on the item to be removed or killed, i.e., microbially decontaminated, by a fluid anti-microbial agent. This is achieved in a variety of ways, including immersing the item in a bath of anti-microbial liquid, spraying the item with anti-microbial liquid, surrounding the item with anti-microbial vapor, and the like. While such systems work well for killing microbes on the exterior surface of the items to be decontaminated, internal lumens can be problematic. To be a viable commercial product, a sterilization or disinfection apparatus must provide assured contact between the anti-microbial agent and the microbes. On items with elongated lumens, such as endoscopes, it is desirable that the anti-microbial fluid assuredly contact all surfaces within the lumen. Typically, this is achieved by pumping or drawing the anti-microbial fluid through the lumen.

Often, endoscopes have a plurality of lumens which may have different cross-sections, length, internal obstructions, and the like. It is advantageous to supply the fluid to different lumens at different pressures. Further, some lumens have multiple openings. Typically, plugs are inserted into or over some of the openings to force the anti-microbial fluid to flow the entire length of the lumen. Often, endoscopes have a lumen which does not need to be sterilized and worse yet, can be damaged by contact with fluids. Further, the lumens have a variety of connector styles, such as screw threads, bayonet pipe connectors, and the like, as well as different diameters.

Typically, the sterilization technicians are given a variety of individual plugs and fittings from which they select the most appropriate plugs and fittings for a specific endoscope to be sterilized or disinfected. Being small parts, they are sometimes lost. The technicians, in many cases, improvise by using another part which appears to work. In other cases, the technicians merely make a mistake in selecting fittings or plugs or in making the connections between the fluid supply, fittings, and lumens. When improper plugs or fittings are used and when improper interconnections are made, the assurance that the anti-microbial agent is contacting all microbes within the lumens is lost.

The fittings and plugs typically connect securely with the structures at the lumen ports. At the surfaces of interconnection, microbes can become trapped between the fittings or plugs and the structures at the lumen port. When there is a good frictional fit, the frictional fit protects these microbes from the antimicrobial agent. This creates the possibility that at the end of the cycle there may be active microbes on the surfaces adjacent the lumen ports destroying the assurance of disinfection or sterility. One solution to the trapped microbe problem is shown in U.S. Patent Nos. 5,552,115 and 5,833,935 of Malchesky in which the fittings and plugs are made of an open-celled plastic material. The porous fitting solution is effective, but does have some drawbacks. First, the porous plastic material is relatively soft. With repeated use, dimensions can change altering flow characteristics. Moreover, the plastic can be damaged or broken during use, again altering flow characteristics. After a disinfection or sterilization cycle, the fittings are typically wet with water from the final rinse. Wet, porous materials can become breeding grounds for airborne microbes if not handled properly. One use, disposable porous connectors and fittings can be costly and there is no assurance that the operator will use a new fitting in each cycle rather than reusing an old one.

The present invention provides a new and improved method and apparatus which overcomes the above-referenced problems and others.

### Summary of the Invention

In accordance with another one of the present invention, a method of disinfection or sterilization of an article having interior lumens is provided. The method includes placing the article in a chamber and interconnecting a lumen port of the article which provides access to its lumen with an antimicrobial fluid outlet. An exterior of the article is contacted with an antimicrobial fluid. The antimicrobial fluid is flowed through the lumen. The method further includes selectively fluidly connecting the anti-microbial fluid outlet and the lumen port of the article with a connector having a fitting which is configured for loose interconnection with surfaces adjacent the lumen port such that a first fraction of the antimicrobial fluid flows through the lumen port into the lumen and a second fraction of the antimicrobial fluid flows between the lumen port and the fitting into the chamber, the interconnection being sufficiently loose that the fitting wobbles, changing momentary points of contact between the fitting and the surfaces adjacent the lumen port.

In accordance with another aspect of the present invention, a fluid disinfection or sterilization system is provided. A chamber receives a lumened article to be microbially decontaminated. A plurality of fluid outlets in the chamber supplies antimicrobial fluid to contact exterior. surfaces of the lumened article. There is at least one second fluid outlet through which the antimicrobial fluid is suppliable to an interior lumen of the lumened article. A connector selectively fluidly connects the second fluid outlet with a lumen port of the article for supplying the antimicrobial fluid to the interior lumen. The connector includes a tube, a first fitting connected to one end of the tube for interconnection with the second fluid outlet, and a second fitting connected with the tube and configured for selective loose interconnection with the lumen port in such a manner that an annular gap forms between the fitting and the port and the fitting wobbles, changing momentary points of contact with surfaces adjacent the lumen port so that a first fraction of the antimicrobial fluid flows through the. lumen port into the lumen and a second portion of the antimicrobial fluid flows between the lumen port and the second fitting into the chamber.

One advantage of the present invention resides in the anti-microbial fluid's assured contact with the surfaces abutting the fittings and plugs.

Another advantage of the present invention is that it promotes the use of the proper fittings and plugs with each endoscope.

Another advantage of the present invention is that it is easy and convenient to use.

Another advantage of the present invention resides in consistent, repetitive operation.

Another advantage of the present invention is that it provides anti-microbial fluid flow through dead-end passages.

Another advantage of the present invention is that it assures that the fittings and plugs are correctly matched to each type of endoscope.

Still further advantages and benefits of the present invention will become apparent to those of ordinary skill in the art upon reading and understanding the following detailed description of the preferred embodiments.

### Brief Description of the Drawings

The invention may take form in various components and arrangements of components, and in various steps and arrangements of steps. The drawings are only for purposes of illustrating preferred embodiments and are not be construed as limiting the invention.
FIGURE 1 is diagrammatic illustration of an exemplary fluid disinfection/sterilization system in accordance with the present invention;
FIGURE 2 is a detailed view of an exemplary tethered fitting and plug assembly in accordance with the present invention;
FIGURE 3 illustrates another tethered plug and fitting assembly;
FIGURE 4 illustrates the interconnection between one of the tethered fittings and a port (shown in phantom) on an endoscope;
FIGURE 5 illustrates another fitting for interconnection between a tube assembly and an endoscope port;
FIGURE 6 is a cross-sectional view of another fitting for interconnection with an endoscope port;
FIGURE 6A is a cross-sectional view of yet another fitting for interconnection with an endoscope port;
FIGURE 7 is an elevational view in partial section of a plug assembly for interconnection with an endoscope port;
FIGURE 8 is a perspective view of yet another plug assembly for interconnection with an endoscope port;
FIGURE 9 is a perspective view of a plug assembly for interconnection with a pair of endoscope ports;
FIGURE 9A is a cross sectional view of the plug assembly of FIGURE 9;
FIGURE 10 is an elevational view of another plug assembly for interconnection with an endoscope port;
FIGURE 11 is a perspective view of yet another plug assembly for interconnection with a pair of adjacent ports of an endoscope;
FIGURE 12 is a perspective view of yet another plug assembly for interconnection with an endoscope port;
FIGURE 13 is a front view of the plug assembly of FIGURE 12;
FIGURE 14 is a side sectional view of the plug assembly of FIGURE 12 connected to an endoscope port;
FIGURE 15 is a side sectional view of yet another fitting for interconnection with an endoscope port;
FIGURE 16 is a top view of another exemplary fluid disinfection/sterilization system in accordance with the present invention; and
FIGURE 17 is a plumbing diagram of the disinfection/sterilization system of FIGURE 16.

### Detailed Description of the Preferred Embodiments

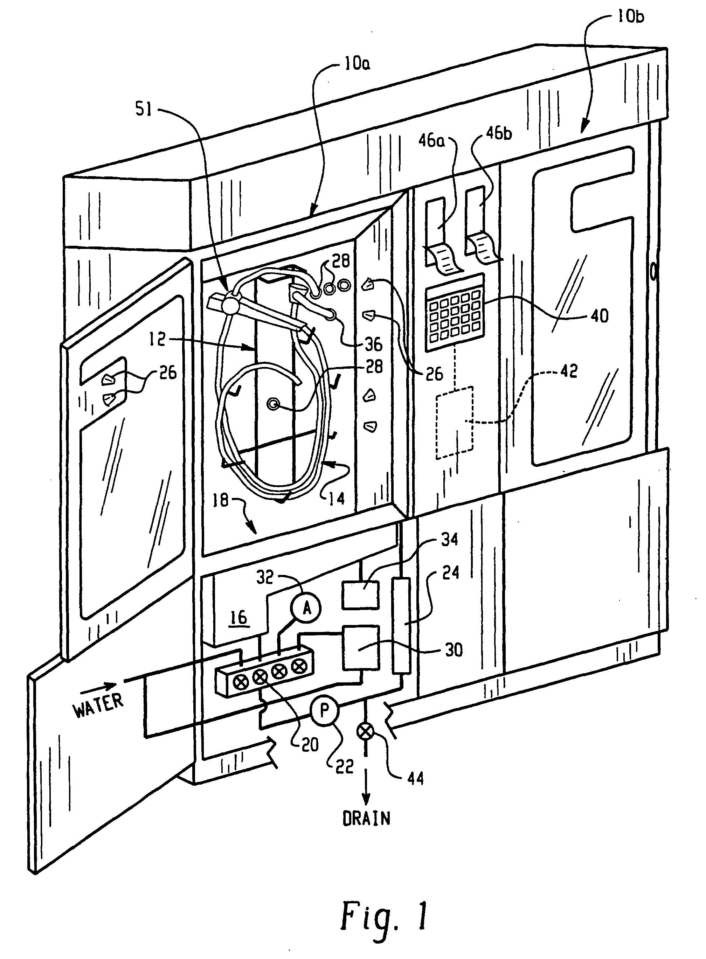

With reference to FIGURE 1, a liquid washing and microbial decontamination system includes a pair of chambers **10a, 10b** for washing and microbially decontaminating endoscopes and other goods. Chamber **10a** is described in detail, but it is to be appreciated that chamber **10b** is analogous. A rack **12** having a plurality of pegs around which the tubes of the endoscope **14** are wound is supported in the chamber. The rack can be hung in the chamber and the endoscope wrapped around it or the endoscope can be wrapped around the rack at a remote location and then the rack and scope are hung as a unit in the chamber. A cup or other ampule containing a washing solution, such as detergent, corrosion inhibitors, and an anti-microbial agent is loaded in a well **16** defined in a lowermost point of a sump **18** at the bottom of the chamber.

A manifold **20** permits any of a plurality of fluids to be connected with a pump **22**. In one state, the manifold connects outside water to the pump **22** which pumps the water through a heater **24** to nozzles **26** located around the chamber and fluid outlet ports **28** located in a rear wall of the chamber. Preferably, some of the ports **28** are high pressure ports and others are low pressure ports. Each of the ports includes a valve that has an open state and a leaky closed state that permits limited fluid flow to assure circulation through the tubing branch leading to it. In another state, the manifold **20** connects the pump with the well **16** at the bottom of the sump to recirculate fluid. In another state, the manifold connects the pump with a sterile water generator **30**. In yet another state, the manifold connects the nozzles (either through the pump or directly) with a source of sterile air **32**, preferably under pressure.

A leak detector **34** is connected with a leak test port 36. The leak detector checks whether a lumen or other structure connected with port **36** is leaking, e.g., whether it holds a preselected vacuum or positive pressure.

It is to be appreciated that analogous elements are connected with the second chamber **10b**, although a common sterile water generator can supply both systems. A common operator input device **40**, such as touch screen, enables the operator to put processing instructions into a common control **42** for the two chambers. The common control causes the leak check device to check whether the lumen connected with the port **36** is leaking or not. The automatic control also controls the manifold **20** and a cup opening device (not shown) in the well **16**, the pump **22**, the heater **24**, and a drain valve **44**. A typical cycle includes pumping cold water to the spray nozzles and the interior lumen ports to remove gross debris, after which the water is drained. Next, a washing solution section of the cup in the well **16** is opened as new water is brought in and circulated to the nozzles and the ports to wash the interior and exterior of the endoscope. After the wash and drain cycle, another rinse cycle removes excess detergent or other washing compounds. After the rinse is drained, air is blown through at least the outlet ports **28** and the interior lumens of the endoscope to remove excess fluid. A corrosion inhibitor compartment of the cup is then opened as additional water is brought into the system. The corrosion inhibitors, buffers, and other components in solution are circulated to the nozzles and output ports. Thereafter, a microbicide portion of the cup is opened to release a microbicide into the circulating solution. After the anti-microbial solution is drained, air again blows excess liquid from the lumens of the endoscope. One or more sterile water rinses follow concluding with a blow out of the water from the lumens. At the end of the cycle, the controller **42** causes an appropriate one of printers **46a**, **46b** to print out a record of the completed sterilization or high level disinfection cycle.

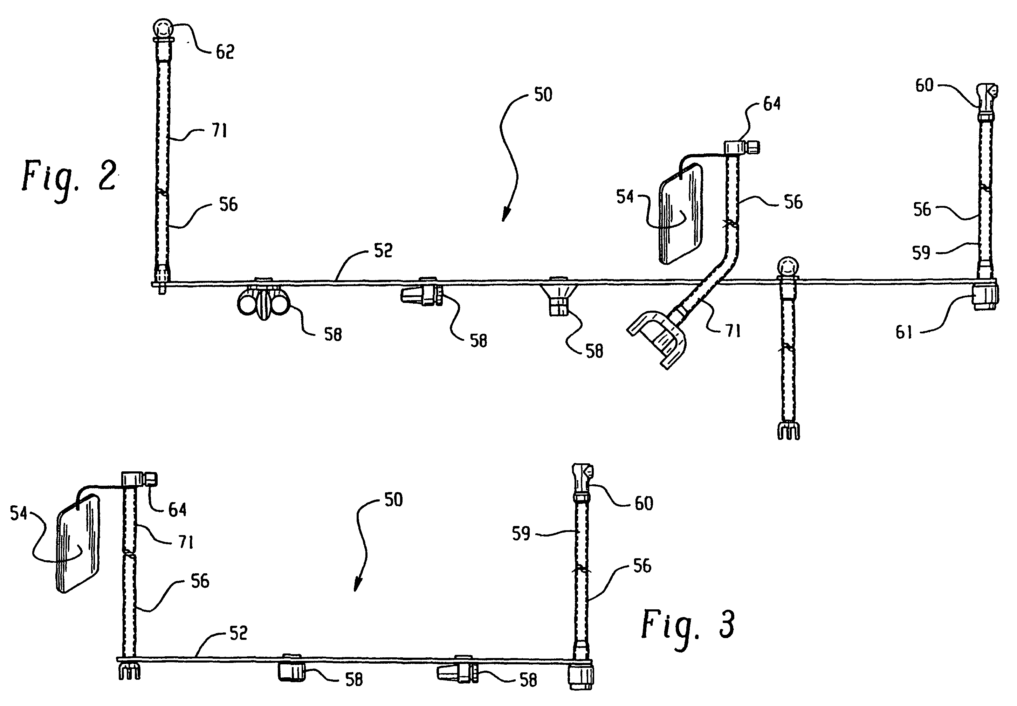

With continuing reference to FIGURE 1, and further reference to FIGURES 2 and 3, after the endoscope 14 is mounted on the rack **12** in the chamber, the operator uses a tethered set of connectors and plugs **50** to interconnect various ports **51** of the endoscope with the liquid ports **28** and the leak detector port **36** and to plug various ports of the endoscope. More specifically, each of the tethered plug and connector assemblies **50** includes a tether **52** which is connected to a tag **54**. The tag **54** carries an indicia of the model or family of models of endoscope which are to be used with tethered set of plugs and connectors. The tag further includes a diagram illustrating how each of the connectors is to be interconnected between the scope and the outlet ports **26** and the leak detector ports. Step by step instructions are also included. Each of the connectors **56** or plugs **58** include a sequential reference character, such as a number or letter, which identify each connector and each plug and correlate the connectors and plugs with the instructions and the order in which they are to be connected.

Typically, one of the connectors **56** includes a tube **59** with a fitting **60** at one end which is configured to mate only with the leak test port **36**. The other end of the connector tube has an appropriate fitting **61** for interconnection with the leak test port of the endoscope. The fluid ports **28** preferably include high pressure ports and low pressure ports. Optionally, the ports may have a larger number of dedicated pressures. Another of the connectors typically has a fitting **62** which is configured to be connected only with one of the high pressure fluid ports **28** and a fitting **74** configured for attachment to an endoscope port structure; while other connectors have a fitting **64** configured to be connectable only with one of the low pressure output ports **28**. Various techniques may be utilized to limit each fitting **62, 64** to be connected with only specific one or ones of the ports **28, 36,** such as different diameters, different connecting mechanisms (threaded, bayonet, etc.), different shapes, and the like. The plugs **58** are each configured to mate with the appropriate ports **51** on the scope identified by the tag. The length of the tether and the length of portions of the tether between the various plugs and connectors are selected such that each of the connectors and fittings just reach a port **51** of the endoscope to which they are to be connected. In this manner, if one of the connectors or plugs is connected with the wrong port, the tether will be too short for other connectors or plugs to reach an available port on the endoscope. This provides a ready indication to the operator that the plugs and fittings have not been connected properly or that the wrong tether assembly has been selected.

While the decontamination system has been described in terms of a liquid system, it will be appreciated that other fluids, such as gaseous or vapor phase antimicrobial compositions may be used in place of the liquids (antimicrobial solution and water rinse) described above. Examples of other fluids include vaporized hydrogen peroxide (a mixture of hydrogen peroxide and water in vapor form), ion plasmas, ethylene oxide, formaldehyde, peracid vapors, such as performic acid, peracetic acid, perpropionic acid, and mixtures thereof.

Additionally, while the decontamination system has been described with reference to spray nozzles and a rack, it is also contemplated that an immersion system may be used in which the endoscope, or other lumened device, is coiled in a receiving tray, or other receptacle, and immersed in the antimicrobial solution. The tray is filled with sufficient antimicrobial solution from one or more fluid outlets to cover the endoscope.

A wide variety of plugs and fittings are connected with the various tethers. Different endoscope manufacturers, and even the same manufacturer within different families of endoscopes, use different types and sizes of port structures. The appropriate fittings **74** and plugs **58** for each of the outlet port structures is preassembled on the tether. Although each of the fittings and plugs is configured to conform with the outlet port structure on the intended endoscope, they are not designed to couple in a fluid-tight connection. To the contrary, the fittings **74** (other than leak test fittings **61**) and plugs **58** are designed to allow limited leakage between their structure and the port structure of the endoscope to which they are mounted. While the fittings **74** and plugs **58** may touch the port structure in some positions at some points, vibration, water flow, and pressure variations cause sufficient movement that the point of contact shifts and all points on the port structure of the endoscope are subject to the anti-microbial fluid during a significant portion of the cycle. Preferably, the nozzles **26** operate in sets. That is, one group of nozzles operates for a while, and then shuts off as another group of nozzles starts operating. This change in spray direction again assists in rocking the fittings **74** and plugs **58** in the associated endoscope port structure.

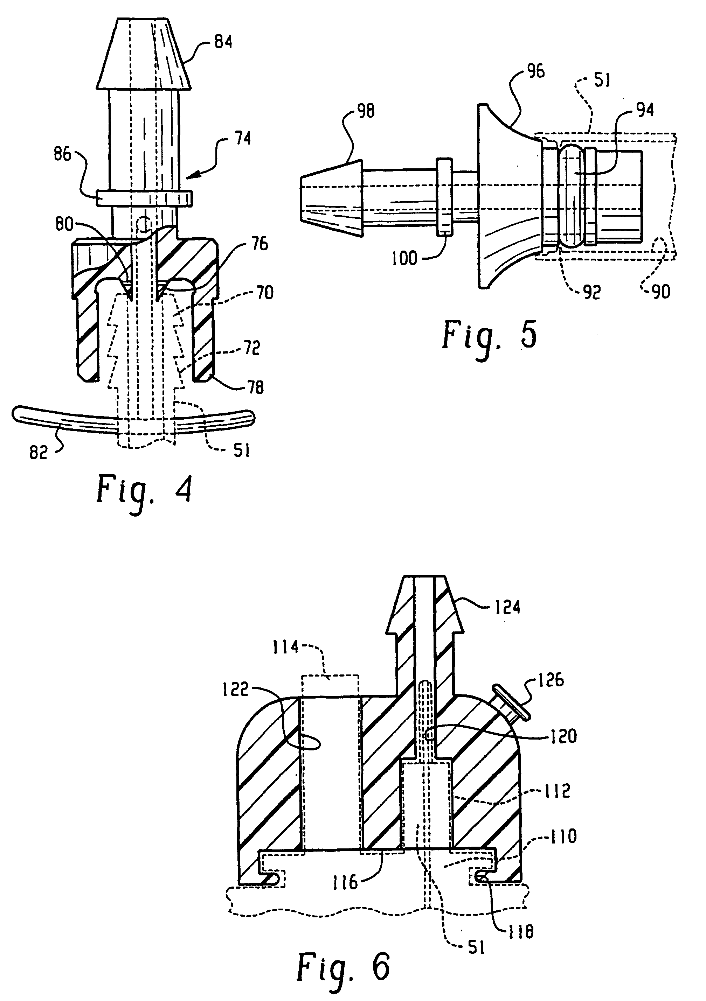

With reference to FIGURE 4, many endoscope ports **51** are defined by extending tubular elements **70** with exterior barbs **72.** A suitable connector **56** for such a port includes a tubular portion **71** with an output port connection fitting **62** or **64** at one end and a fitting **74** adapted to connect to the port at the other. The tubular portion may be branched to allow for more than one fitting **74** on the tubular portion. The fitting **74** includes a body portion that defines a beveled annular ring **76** designed to be engaged partially into the interior of the tubular element **70**. A plurality, e.g., four peripheral leg members **78** surround and are spaced from the exterior of the barbs to maintain alignment and prevent excessive tipping. A plurality of small passages **80** cause a small amount of fluid to be ejected under relatively high pressure and flow over the barbs **72**. Additional fluid flows between the beveled surface **76** and the tubular element **70**. A wire bail **82** is dimensioned to pass under the last of the barbs **72**. The distance between the wire bail and beveled surface **76** is selected to be just slightly longer than the corresponding distance on the fitting such that an annular gap forms between the beveled surface **76** and the port and between the bail and the barbs, although both will make contact from time to time. The housing body further includes a barbed element **84** for interconnection with tubing of the appropriate length for interconnection with the fluid ports. Again, the tubing just reaches the appropriate fluid port **28** to provide an indication that it has been properly connected to the scope. A collar element **86** provides a stop for the tubing and provides a detent over which the tether **52** is fit.

With a reference to FIGURE 5, some endoscope port structures include a tubular segment or internal bore **90** which has a small inward projecting lip or detent **92**. A housing body includes an annular groove in which a C-ring **94** is loosely retained. The C-ring is sufficiently spaced from the body that it can be compressed as it snaps past the lip **92**. Preferably, the C-ring spans about 300º of arc. The C-ring and a shoulder portion **96** of the body are spaced further than the thickness of the lip such that there is in and out play between the tube or bore **90** and the fitting. The fitting further includes a barbed tubular element **98** for interconnection with a length of tubing. A shoulder **100** provides a stop for the tubing and a detent over which the tether **52** is received.

In an alternative embodiment, the C-shaped ring **94** is replaced with an annular ridge, which is integral with the adapter body.

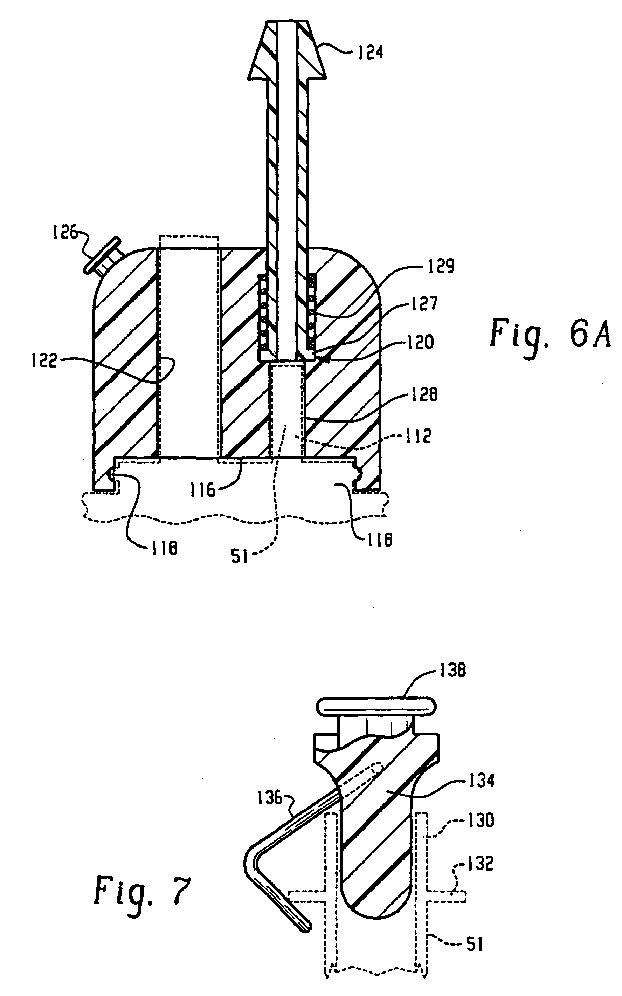

With reference to FIGURE 6, some endoscopes have raised port structures **110** supporting both a tubular structure **112** and a post **114**. The fitting **74** includes a fitting body having a lower surface **116** and inwardly projecting detents or arc segments **118** for snapping or twisting under a lip on the structure **110**. The spacing between the bottom surface **116** and the detents **118** is again slightly larger than the thickness of the lip of the raised portion **110** to provide a thin fluid flow path therebetween. The body further defines a bore **120** which is dimensioned just larger than the tubular pipe 112 such that most of the fluid flow flows down its bore. A small amount flows around the periphery. A second bore 122 is again slightly larger in diameter than the post 114 to form a narrow angular cap therebetween. A portion of the fluid flowing between the bottom surface of housing and the mounting element 110 flows through the bore. Again, the dimensions are sufficiently loose that the fitting is movable short distances longitudinally and along canting directions. The fitting again includes a barb 124 for interconnection with associated tubing and a post 126 for interconnection with the tether 52.

With reference to FIGURE 6A, a modification to the fitting 74 of FIGURE 6 is shown. The fitting 74 of FIGURE 6A includes a fitting body having a lower surface 116 and inwardly projecting detents or arc segments 118 for snapping or twisting under a lip on the structure 110. The spacing between the bottom surface **116** and the detents **118** is again slightly larger than the thickness of the lip of the raised portion **110** to provide a thin fluid flow path therebetween. The body further defines a bore **120** which is dimensioned to receive the tubular pipe **112.** A second bore **122** is again slightly larger in diameter than the post 114 to form a narrow angular cap therebetween. A portion of the fluid flowing between the bottom surface of housing and the mounting element **110** flows through the bore. Again, the dimensions are sufficiently loose that the fitting is movable short distances longitudinally and along canting directions. The fitting again includes a barb **124** for interconnection with associated tubing and a post **126** for interconnection with the tether **52.** The barb, in this embodiment, takes the form of a moveable plunger. A widened proximal end **127** of the barb **124** is received within the bore **120** and biased toward a narrowed portion **128** of the bore by a compression spring **129**, or other biasing member. The leak rate is controlled by the force of the spring on the plunger. Under the force of the fluid flowing through the barb, the plunger is pushed slightly outward, away from the narrow portion of the bore, allowing the fluid to leak between the outside of the tube 112 and the narrow portion of the bore.

With reference to FIGURE 7, some ports are defined by tubular elements **130** having an annular collar **132**. When it is appropriate to plug these ports, one type of plug **58** includes a housing body that tapers into an extension **134** slightly smaller in diameter than the interior bore of the tubular element **130.** A wire bail **136** is pivotally connected to the body to snap under the collar **132**. Again, the dimensions are such that during normal vibration, fluid flows between the plug and the tube and fluid flows between wire bail **136** and the collar **132**. An enlarged portion **138** is again provided for receiving the tether **52**.

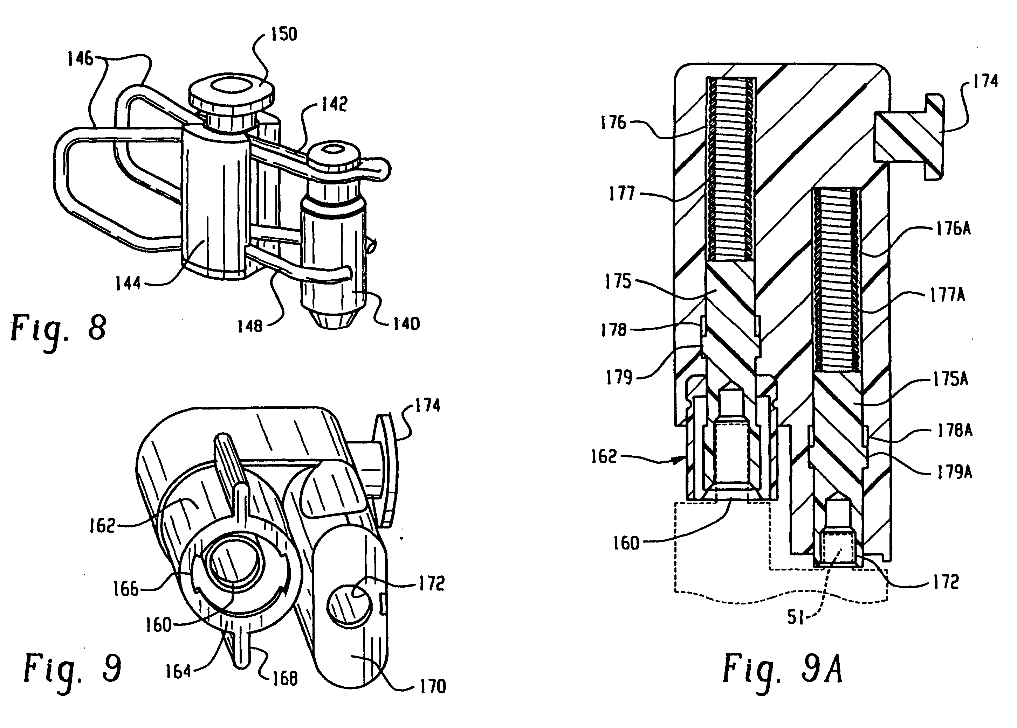

Reference to FIGURE 8, for easier connection and disassembly, a plug **58** includes a plug element **140** which is slightly smaller in diameter than the bore of the port to be plugged. The plug element is connected by wire member **142** with a body portion **144**. A pair of wire handles **146** are pivotally connected through the body portion with a pair of wires to form gripping elements which engage a groove in or under the underside of a lip surrounding the port. In this manner, by squeezing and releasing the handles **146**, the plug can be inserted into the port and wire spring elements **148** can hold it loosely in place. A button **150** provides a convenient interconnection with the tether **52**.

With reference to FIGURE 9, some ports are surrounded by a tubular element having a pair of outward detents for a bayonet type interconnection. A plug housing body includes a tapered annular surface **160** analogous to surface **76** of FIGURE 4. The body further includes a rotatable portion **162** having an inward directed flange **164** with a pair of cutouts **166** for receiving the projecting detents on the port. After the detents are received through the cutout portion **166**, the operator engages a handle portion **168** and rotates rotatable portion **162** by a quarter turn to lock it in place over the fitting. Again, the dimensions are such that the plug 58 wobbles sufficiently to provide flow over all surfaces. In some endoscopes, a projecting tubular element is disposed adjacent the other port. To this end, the housing body further includes a section **170** having a bore **172** of just slightly larger diameter than the tube to be received. Where appropriate, an internal bore extends between the bore **172** and the interior of the conical surface 160 to provide a controlled fluid flow path between the two ports. A button **174** provides a convenient connection point for the tether.

With reference to FIGURE 9A, in a modified embodiment of FIGURE 9, the plug housing body includes a pair of spring loaded plungers **175**, **175A** received in bores **176**, **176A**. The plungers define the tapered annular surface **160** and bore **172**, respectively, which receive the endoscope ports. As for FIGURE 9, a rotatable portion **162** having an inward directed flange with a pair of cutouts receives the projecting detents on the port and is rotated to lock the plug assembly to the port. The plungers are biased towards open ends of the bores **175** and **175A** by biasing members, such as springs **177**, **177A**. The plungers have limited vertical motion, defined by widened portions **178**, **178A** of the bores and corresponding widened potions of the plungers **179**, **179A**. Under the pressure of the fluid entering the tapered annular surface **160** and bore **172**, the plungers move upwards slightly, away from the endoscope ports, and allow the fluid to leak around the sides of the endoscope port. The rate of leakage can be adjusted by changing the tension force supplied by the spring. In one embodiment, the two springs provide different forces so that fluid leaks preferentially from one of the ports.

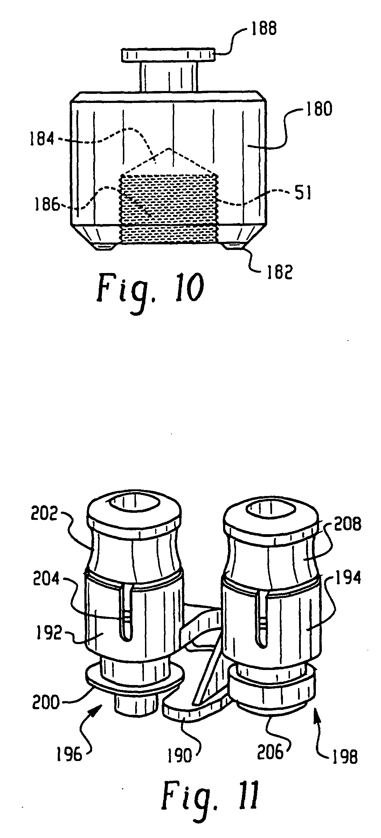

With reference to FIGURE 10, a plug **58** includes a housing body **180** having two projecting feet **182** to hold the plug away from an associated surface of the endoscope. The plug body has an interior bore **184** that is internally threaded with non-sealing threads **186**. NPT threads are designed for a fluid tight seal, but other standard threads, e.g., acme threads, are not. The threads are redimensioned such that they leave gaps as they loosely engage the threads of the scope port. Wobble between the threads provides changing fluid flow paths through the threaded connection. Optionally, sections of the threads may be removed to create an enlargement **186** in one more locations down the side of the internal bore to provide for less restrictive fluid flow. The contact points **182** prevent the threads from being screwed down so tight that fluid is not permitted to flow between the bottom of the housing and the endoscope, and preventing closing of the gaps between the threads of the fitting and the threads of the endoscope. A button **188** provides a convenient mounting point for the tether **52**.

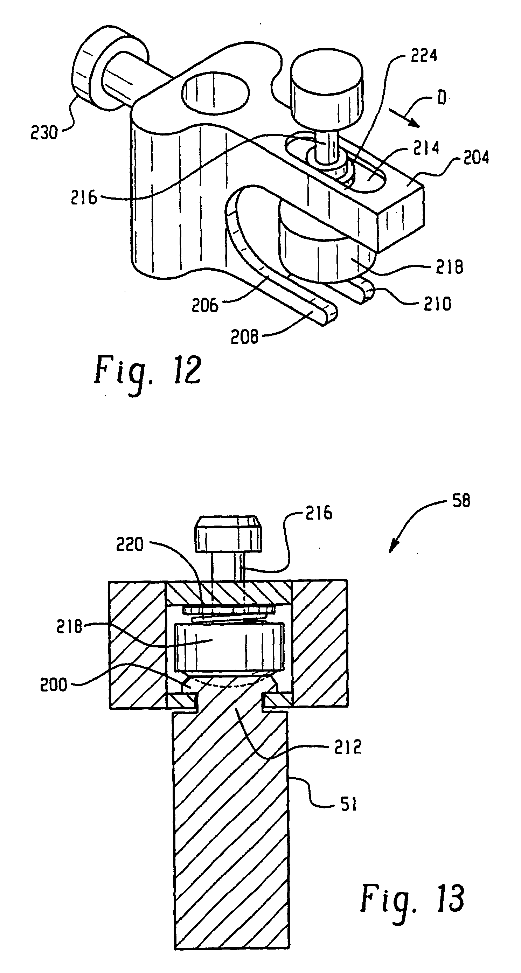

With reference to FIGURE 11, on some endoscopes there are pairs of ports to be plugged. In the embodiment of FIGURE 11, the housing body of the plug **58** includes a lower tab **190** which slides under outward extending lips on a pair of tubes on an associated structure. The body defines a pair of cylinders **192, 194** in which plungers **196, 198** are mounted. The plunger **196** is smaller in diameter than the tube to be plugged with a surrounding flange 200 of a diameter a little smaller than the internal diameter of the bore in which it is received. A spring (not shown) within the housing **192** biases the plug into the opening. A handle portion **202** enables the plug to be pulled up against the biasing portion of the spring. When pulled up and rotated, a detent **204** moves out of the corresponding slot to hold the plunger retracted. The plunger 198 has a beveled lower edge **206** which is biased against the surrounding edge of the scope port by a spring (not shown) in the housing **194**. A handle **208** again enables the plunger **198** to be retracted and, when turned, held retracted. In this manner, the operator retracts both plungers and slides the tab **190** underneath surrounding lips. The two plungers are then released under the spring bias. The plunger handles **202** and **208** are dimensioned such that, upon release, they stop on the housing cylinders **192** and **194** before the plungers **196** and **198** contact the device, leaving a gap which allows fluid to flow around the port.

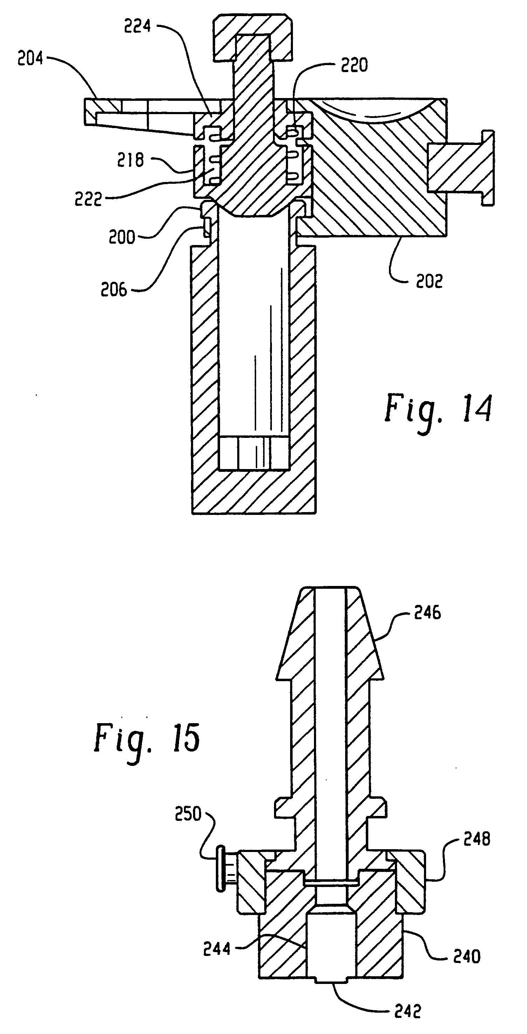

With reference to FIGURES 12-14, a plug **58** suited to use with an endoscope port having a flange **200** at its open end is shown. The plug includes a housing body **202** having an upper arm **204** and a shorter, U-shaped lower arm **206**. The flange **200** of the endoscope port is seated on legs **208**, **210** of the U-shaped lower arm with a narrow portion **212** of the endoscope port positioned between the two legs. The upper arm defines a longitudinal slot **214** which receives a shaft **216** therethrough. A lower end of the shaft defines a plug element **218** which plugs the opening of the endoscope port. The plug element is biased toward the port by a spring **220** or other biasing element received in an annular groove **222** of the plug element (FIGURE 14). The spring is held under tension by a disk-shaped member **224** which is carried on the shaft between the plug element and the upper arm. To fit the plug on the endoscope, the shaft is pushed along the slot in the direction of arrow D until the shaft reaches the end of the slot **214**. The plug element is then seated on the endoscope port. The housing body **202** is then pushed toward the port until the flange **200** engages the U-shaped arms. Pressure may be applied to the upper arm during this operation for ease of attachment. The spring forces the plug into engagement with the opening. Under the pressure of fluid within the port, the plug element is biased away from the port, leaving a small annular gap through which the fluid leaks from the port. A button **230** provides a convenient mounting point for the tether **52**.

With reference to FIGURE 15, a fitting has a housing body **240**, similar to housing body **180** of FIGURE 10, and has two projecting feet **242.** An interior bore **244** is internally threaded with non-sealing threads that leave gaps as they loosely engage the threads of the endoscope port. Wobble between the threads provides changing fluid flow paths through the threaded connection. A barb **246** for attachment to a tube **71** is connected with the housing by a thumb wheel **248**, which is free to spin relative to the housing. This allows the tube to remain untwisted while the fitting is being connected with an endoscope port. A button **250** provides a convenient mounting point for the tether **52**.

The above discussed fittings and plugs are exemplary only. Numerous additional leaky connections are contemplated. New and improved endoscopes are introduced regularly. The new and improved endoscopes in many instances will have different port configurations which require modifications to the foregoing exemplary fittings and plugs.

The clearance between the plug and the surrounding structure on the endoscope also varies with the degree of stoppage or leakage that is appropriate to the application. In some situations, it is desirable to allow the plug to pass a sufficient amount of fluid that the pressure downstream in the lumen is reduced to a preselected fraction of the upstream pressure. When such a pressure reduction is desired, the clearances between the plug and the endoscope are increased. Optionally, the plug may have a controlled leakage or feedback port or passage.

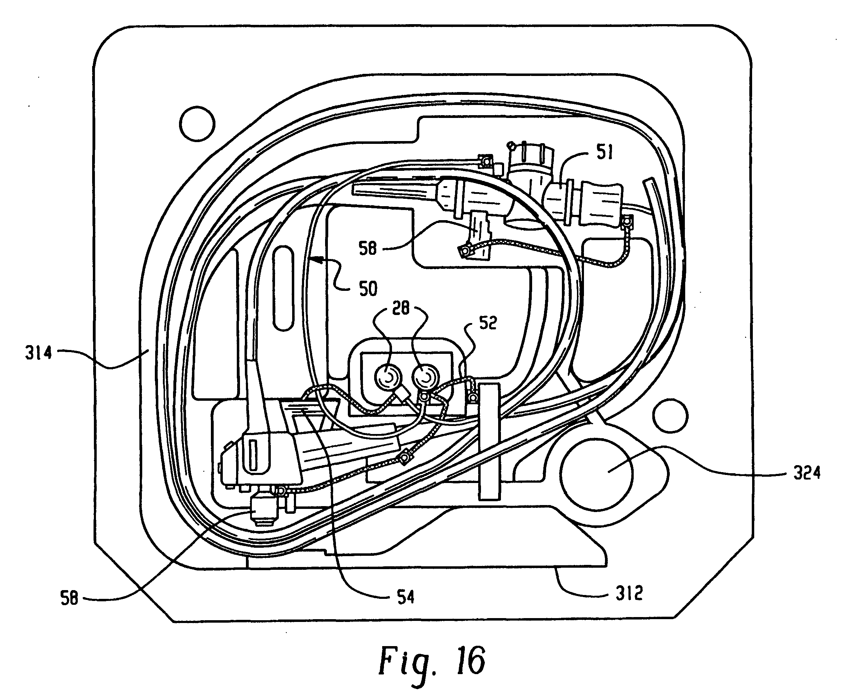

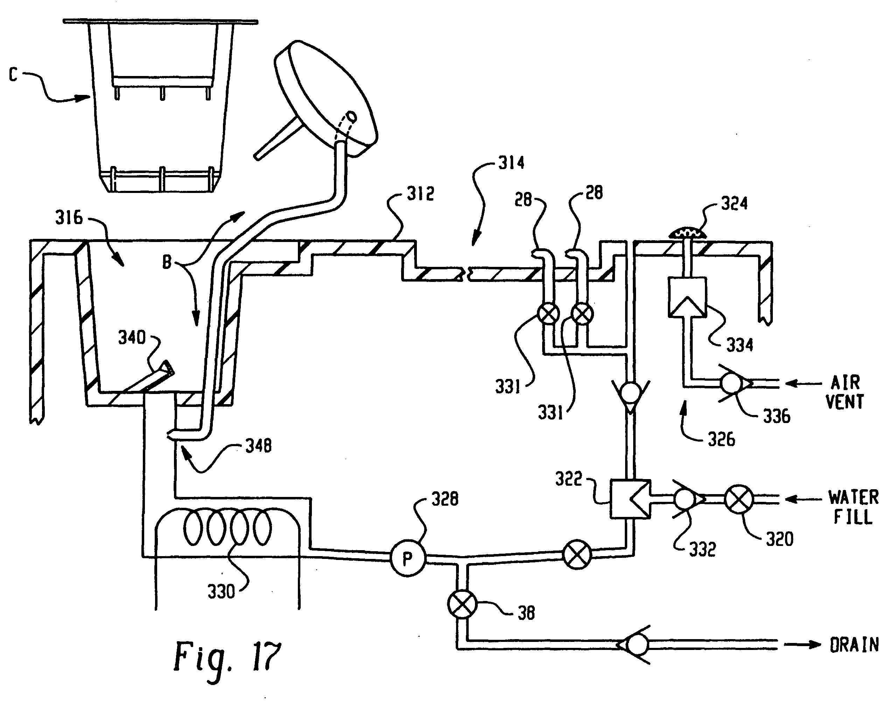

With reference to FIGURES 16 and 17, another embodiment of a microbial decontamination apparatus is configured to sit on a counter top or other convenient work surface. In this embodiment, an endoscope to be microbially decontaminated is fully immersed in a sterilant/disinfectant solution, rather than being sprayed with the solution. A door or lid (not shown) is manually openable to provide access to a tray **312** which defines a receiving region **314** for receiving items to be microbially decontaminated. In the illustrated embodiment, the tray **312** is configured to receive an endoscope or other long, coilable item. Other trays with item receiving regions of different configurations for receiving the items themselves or item holding containers are also contemplated. A well **316** receives a cup C containing a unit dose of reagents for forming a sterilant, disinfectant, or other microbial decontaminating solution.

A tethered set **50** of connectors and plugs adapted to the particular endoscope to be decontaminated is interconnected with various ports **51** of the endoscope and with a liquid supply port or ports **28** within the tray or adjacent thereto in a similar manner to that described for the spray decontamination system.

With particular reference to FIGURE 17, a reagent containing package **C** is inserted into the well **316**. Once the items are loaded into the tray and the reagent carrying package **C** is inserted into the well **316**, the lid **310** is closed and latched. Optionally, a fill valve **320** passes water through a microbe removing filter **322** in flow paths of a fluid circulating system. The microbe removing filter **322** provides a source of sterile water by passing water and blocking the passage of all particles the size of microbes and larger. The incoming water which has been sterilized by the filter **322** passes through a spray or distribution nozzle **324** and fills the item receiving region **314** in the tray **312**. The water may also be passed through the leaking connectors to the internal endoscope passages. As additional water is received, it flows into the well **316** dissolving powdered, crystalline, or other non-liquid reagents in the cup **C**, forming an anti-microbial solution. Filling is continued until all air is forced through an air system **326** and an entire interior volume is filled with the sterile water. After the fill valve **320** is closed, a pump **328** circulates the fluid through a heater **330**, the item receiving region **314** of the tray **312**, and the well **316**. The pump also supplies the fluid under pressure via appropriate, separately adjustable presure regulators or valves **331** to the port(s) **28** and hence to the internal passages of the endoscope via the tethered connectors **56**, while the plugs **58** inhibit entry of the circulating fluid in the tray to those ports with which they are connected. Because of the difference in fluid pressure between the interior passages of the endoscope and the tray, there is some fluid leakage out of the endoscope via the leaking plugs. The pump also forces the anti-microbial solution through the filter **322** to a check valve **332** sterilizing the filter. Further, the pump forces the anti-microbial solution through another microbe filter **334** in the air system **326** to a check valve **336**. After the anti-microbial solution has been brought up to temperature and circulated for a selected duration, a drain valve **338** is opened, allowing the solution to drain. Air is drawn through the microbe filter **334** such that sterile air replaces the fluid within the system. Thereafter, the drain valve is closed and the fill valve **320** opened again to fill the system with a sterile rinse fluid. It will be noted, that because the pump **328** circulated the anti-microbial solution over all surfaces of the flow paths including all surfaces leading from the sterile rinse source **322**, the rinse cannot bring microbial contaminants into the item receiving region **314**. Sterile rinse fluid is fed to the internal passages of the endoscope via the leaking connectors **56**.

A cup opener **340** is disposed at the bottom of the well for engaging a lower surface of the package **C** as it is inserted into the well.

## Claims

1. A method of disinfection or sterilization of an article (14) having interior lumens, the method comprising placing the article in a chamber (10a, 10b), interconnecting a lumen port (51) of the article which provides access to its lumen with an antimicrobial fluid outlet (28), contacting an exterior of the article with an antimicrobial fluid and flowing the antimicrobial fluid through the lumen, the method comprising the step of
fluidly interconnecting. the anti-microbial fluid outlet and the lumen port of the article with a connector (56) having a fitting (74) which is configured for loose interconnection with surfaces adjacent the lumen port such that a first fraction of the antimicrobial fluid flows through the lumen port into the lumen and a second fraction of the antimicrobial fluid flows between the lumen port and the fitting into the chamber, the interconnection being sufficiently loose that the fitting wobbles, changing momentary points of contact between the fitting and the surfaces adjacent the lumen port.

2. The method as set forth in claim 1, further **characterized by**:
the article having a plurality of lumens, and the connector (56) being connected by a tether (52) to at least one of (i) another connector (56) and (ii) a plug (58), the method further comprising:
connecting each of the connectors between an antimicrobial outlet (28) and a lumen port (51); and
connecting each of the plugs (58) with lumen ports (51).

3. The method as set forth in claim 2, further **characterized by**:
the article to be microbially decontaminated being an endoscope.

4. The method as set forth in claim 3, further **characterized by**:
attaching a tag (54) to one of the tether (52), connector (56), and plug, which identifies at least one of (i) a model of endoscope or (ii) a family of endoscope models with which the tethered connection assembly is to be utilized.

5. The method as set forth in claim 4, further **characterized by**:
the tag (54) including:
a diagram illustrating proper interconnection of the tethered connectors and plugs with the fluid outlets and the lumen ports.

6. The method as set forth in any one of preceding claims 2-5, further **characterized by**:
each of the connectors and plugs including an indicia of interconnection order and being attached to the tether in accordance with the interconnection order, the method further including:
attaching the connectors and plugs to their corresponding lumen ports in the order indicated.

7. The method as set forth in any one of preceding claims 2-6, further **characterized by**:
the plugs and connectors being interconnected with the tether in an appropriate order and spacing along the tether such that the tether allows each of the plugs and connectors to just reach a corresponding lumen port on the article while not reaching sufficiently far for at least some of the connectors and plugs to reach non-corresponding lumens on the article.

8. A fluid disinfection or sterilization system comprising a chamber (10a, 10b) for receiving a lumened. article (14) to be microbially decontaminated, a plurality of fluid outlets (26, 324) in the chamber for supplying antimicrobial fluid to contact exterior surfaces of the lumened article and at least one fluid outlet (28) through which the antimicrobial fluid is suppliable to an interior lumen of the lumened article, the system comprising:
a connector (56) for fluidly connecting said fluid outlet with a lumen port (51) of the article for supplying the antimicrobial fluid to the interior lumen, the connector including:
a tube (71);
a first fitting (62, 64) connected to one end of the tube for interconnection with said fluid outlet ; and
a second fitting (74) connected with the tube and configured for loose interconnection with the lumen port in such a manner that an annular gap forms between the fitting and the port and the fitting wobbles, changing momentary points of contact with surfaces adjacent the lumen port so that (i) a first fraction of the antimicrobial fluid flows through the lumen port into the lumen and (ii) a second fraction of the antimicrobial fluid flows between the lumen port and the second fitting into the chamber.

9. The system as set forth in claim 8, further **characterized by**:
said fluid outlet (28) including a closure valve which has an open state and a leaky closed state that substantially but not completely closes said fluid outlet port when not interconnected with a fitting (62,64).

10. The system as set forth in any one of claims 8-9, further **characterized by**:
a plug (58) configured for loose interconnection with a second lumen port (51) in such a manner that a fraction of antimicrobial fluid flowing through the lumen flows out of the second lumen port, between the plug and the port.

11. The system as set forth in claim 10, further **characterized by**:
a tether (52) which interconnects the connector and at least the plug (58), whereby the connector (56) and the plug remain tethered together as a tethered assembly (50).

12. The system as set forth in any one of claims 8-10, further **characterized by**:
at least a second connector (56); and
a tether (52) which interconnects the first connector (56) and the at least a second connector (56), whereby the first and second connectors remain tethered together as a tethered assembly (50).

13. The system as set forth in any one of claims 11 and 12, further **characterized by**:
the chamber further including a port (36) interconnected with a leak detector (34); the article having a leak test port;
a tube assembly (56) having a fitting (60) at one end configured for interconnection only with a port (36) of the leak detector and another fitting (61) at an opposite end configured for interconnection with a leak test port of the article, the tether tethering the tube assembly and the connector together.

14. The system as set forth in any one of claims 8-13, further **characterized by**:
the article to be microbially decontaminated being an endoscope.

15. The system as set forth in claim 14, further **characterized by** the tethered connection assembly further including:
a tag (54) which identifies at least one of (i) a model of endoscope or (ii) a family of endoscope models with which the assembly is to be utilized, the tag being connected to the tether.

16. The system as set forth in claim 15, further **characterized by**:
the tag (54) further including:
a diagram illustrating proper interconnection of the tethered connectors and plugs with the lumen ports of the endoscope.

17. The system as set forth in any one of claims 12-16, further **characterized by**:
the tethered assembly including a plurality of connectors and a plurality of plugs, all tethered together by the tether.

18. The system as set forth in claim 17, further **characterized by**:
each of the connectors and plugs including an indicia of interconnection order and being attached to the tether in accordance with the interconnection order.

19. The system as set forth in claim 17, further **characterized by**:
the article including a plurality of lumen ports;
each of the plugs and connectors being configured for being interconnected with a corresponding one of the lumen ports;
the plugs and connectors being interconnected with the tether in an appropriate order and spacing along the tether such that the tether allows each of the plugs and connectors to just reach the corresponding lumen port on the article while not reaching sufficiently far for at least some of the connectors and plugs to reach non-corresponding lumen ports on the article.

20. The system as set forth in any one of preceding claims 8-19, further **characterized by**:
the first fluid outlet (26) including spray nozzles which are operated in at least two groups facilitating movement between the fittings and plugs and and corresponding lumen ports.

21. The system as set forth in any one of preceding claims 8-19, further **characterized by**:
a receiving tray (312) which receives the article, the first fluid outlet (324) supplying fluid to the tray.

## Patentansprüche

1. Ein Methode zur Desinfizierung oder Sterilisation eines Gegenstands (14) mit inneren durchgehend offenen Hohlräumen (Lumen), wobei die Methode das Einbringen des Gegenstands in eine Kammer (10a, 10b) umfasst und des weiteren das Verbinden des Lumen-Einlassstutzens (51) des Gegenstands mit einem Mikrobizid-Füllstutzen (28), wobei das Mikrobizid über das Äußere des Gegenstands laufen und durch das Lumen des Gegenstands hindurchfließen soll, und besagte Methode den Schritt umfasst, dass
der Auslassstutzen der mikrobiziden Flüssigkeit mit dem Lumen-Einlassstutzen des Gegenstands mittels einer Schlauchverbindung (56) verbunden wird, deren Fitting (74) so gestaltet ist, dass eine lockere Verbindung mit den sich direkt neben dem Lumen-Einlassstutzen befindlichen Flächen hergestellt wird, so dass eine erste Teilmenge der mikrobiziden Flüssigkeit durch den Lumen-Einlassstutzen in das Lumen und eine zweite Teilmenge der mikrobiziden Flüssigkeit zwischen Lumen-Einlassstutzen und Fitting in die Kammer fließt, und dass diese Verbindung hinreichend locker ist, dass das Fitting lose wackelt und sich dadurch der momentane Kontaktpunkt zwischen Fitting und den sich direkt neben dem Lumen-Einlassstutzen befindlichen Fläche wiederholt ändert.

2. Die Methode gemäß Anspruch 1, des weiteren **dadurch gekennzeichnet, dass**
der Gegenstand eine Vielzahl von Lumen besitzt, und die Schlauchverbindung (56) über einen Verbindungsstrang (52) zumindest (i) mit einer weiteren Schlauchverbindung (56) und (ii) mit einem Stöpsel (58) verknüpft ist, wobei die Methode des weiteren Folgendes umfasst:
das Verbinden einer jeden der Schlauchverbindungen zwischen einem Mikrobizid-Füllstutzen (28) und einem Lumen-Einlassstutzen (51); und
das Verbinden eines jeden der Stöpsel (58) mit jeweils einem Lumen-Einlassstutzen (51).

3. Die Methode gemäß Anspruch 2, des weiteren **dadurch gekennzeichnet, dass**
der mikrobiell zu dekontaminierende Gegenstand ein Endoskop ist.

4. Die Methode gemäß Anspruch 3, des weiteren **dadurch gekennzeichnet, dass**
eine Marke (54) an dem Verbindungsstrang (52), an der Schlauchverbindung (56) oder an dem Stöpsel (58) angebracht ist, durch die wenigstens (i) das Model des Endoskops oder (ii) die Familie von Endoskop-Modellen identifiziert wird, bei dem oder bei der die strangverbundene Einheit eingesetzt werden kann.

5. Die Methode gemäß Anspruch 4, des weiteren **dadurch gekennzeichnet, dass**
die Marke (54) Folgendes enthält:
eine schematische Darstellung, die den korrekten Anschluss der an dem Verbindungsstrang angebrachten Schlauchverbindungen und Stöpsel an die Mikrobizid-Füllstutzen und Lumen-Einlassstutzen darstellt.

6. Die Methode gemäß eines beliebigen der vorhergehenden Ansprüche 2 bis 5, des weiteren **dadurch gekennzeichnet, dass**
jede der Schlauchverbindungen und Stöpsel eine Markierung bezüglich der einzuhaltenden Reihenfolge bei der Herstellung der Verbindung trägt und auch in dieser Reihenfolge an dem Verbindungsstrang angebracht ist, und die Methode des weiteren umfasst,
dass die Schlauchverbindungen und Stöpsel mit den ihnen entsprechenden Lumen-Anschlussstutzen in besagter Reihenfolge verbunden werden.

7. Die Methode gemäß eines beliebigen der vorhergehenden Ansprüche 2 bis 6, des weiteren **dadurch gekennzeichnet, dass**
die Stöpsel und Schlauchverbindungen an dem Verbindungsstrang in entsprechender Reihenfolge und dabei entlang dem Verbindungsstrang so weit auseinander angebracht sind, dass jeder der Stöpsel und Schlauchverbindungen gerade den ihm oder ihr entsprechenden Lumen-Einlassstutzen an dem Gegenstand erreicht, und dass dabei zumindest einige der Stöpsel und Schlauchverbindungen nicht an irgendwelche Lumen des Gegenstandes heranreichen können, die ihnen nicht entsprechen.

8. Ein auf Flüssigkeiten basierendes Desinfektions- oder Sterilisiersystem bestehend aus einer zur Aufnahme des mikrobiell zu dekontaminierenden Lumen enthaltenden Gegenstands (14) geeigneten Kammer (10a, 10b), einer Vielzahl von in die Kammer führenden Füllstutzen (26, 324) zur Versorgung der mikrobiziden Flüssigkeit, die über die äußere Flächen des Lumen enthaltenden Gegenstands spült, und zumindest einen Mikrobizid-Füllstutzen (28), durch den die mikrobizide Flüssigkeit in das Innere eins Lumens des Lumen enthaltenden Gegenstands zugeführt werden kann, und besagtes System Folgendes enthält:
eine Schlauchverbindung (56) zum Anschließen des besagten Mikrobizid-Füllstutzens an den Lumen-Einlassstutzen (51) des Gegenstands, durch die sodann das Mikrobizid in das Lumen eingefüllt werden kann, wobei besagte Schlauchverbindung das Folgende enthält:
einen Schlauch (71);
ein erstes, an dem einen Ende des Schlauchs montiertes Fitting (62, 64) zum Anschließen der besagten Schlauchverbindung an den Mikrobizid-Füllstutzen; und
ein zweites, an dem Schlauch montiertes Fitting (74), das so ausgelegt ist, dass damit eine lockere Verbindung mit dem Lumen-Einlassstutzen derart hergestellt werden kann, dass dabei ein ringförmiger Spalt zwischen dem Fitting und dem Einlassstutzen entsteht und das Fitting lose wackelt und sich dadurch der momentane Kontaktpunkt zwischen Fitting und den sich direkt neben dem Lumen-Eintassstutzen befindlichen Fläche wiederholt ändert, so dass (i) eine erste Teilmenge der mikrobiziden Flüssigkeit durch den Lumen-Einlassstutzen in das Lumen und (ii) eine zweite Teilmenge der mikrobiziden Flüssigkeit zwischen Lumen-Einlassstutzen und zweitem Fitting in die Kammer fließt.

9. Das System gemäß Anspruch 8, des weiteren **dadurch gekennzeichnet, dass**
besagter Füllstutzen (28) ein Abschlussventil enthält, das eine offenen Stellung hat sowie eine leckende Schließstellung, in der besagter Füllstutzen zum Großteil aber nicht vollständig geschlossen ist, wenn er nicht mit einem Fitting (62, 64) verbunden ist.

10. Das System gemäß eines beliebigen der vorhergehenden Ansprüche 8 und 9, des weiteren **dadurch gekennzeichnet, dass**
es einen Stöpsel (58) enthält, der so ausgelegt ist, dass damit ein lockerer Abschluss derart hergestellt werden kann, dass eine Teilmenge der durch das Lumen fließenden mikrobiziden Flüssigkeit aus dem zweiten Lumen-Einlassstutzen zwischen Stöpsel und Stutzen heraus fließt.

11. Das System gemäß Anspruch 10, des weiteren **dadurch gekennzeichnet, dass** System Folgendes enthält:
einen Verbindungsstrang (52), der die Schlauchverbindung (56) und zumindest den Stöpsel (58) miteinander verbindet, und damit die Schlauchverbindung und der Stöpsel als strangverbundene Einheit (50) zusammengehalten bleiben.

12. Das System gemäß eines beliebigen der vorhergehenden Ansprüche 8 bis 10, des weiteren **dadurch gekennzeichnet, dass** das System Folgendes enthält:
zumindest eine zweite Schlauchverbindung (56); und
einen Verbindungsstrang (52) der die erste Schlauchverbindung (56) mit zumindest einer zweiten Schlauchverbindung (56) verbindet, und dadurch die erste und zweite Schlauchverbindung als strangverbundene Einheit (50) zusammengehalten bleiben.

13. Das System gemäß eines beliebigen der vorhergehenden Ansprüche 11 und 12, des weiteren **dadurch gekennzeichnet, dass**
die Kammer einen Stutzen (36) enthält, an dem ein Leckage-Detektor (34) angeschlossen ist;
der Gegenstand mit einem Leckage-Prüfstutzen ausgerüstet ist;
die Schlauchverbindung (56) an einem Ende mit einem, nur an den Stutzen (36) des Leckage-Detektors passenden Fitting (60), und an seinem anderen Ende mit einem, nur an den Leckage-Prüfstutzen des Gegenstands passenden Fitting (61) versehen ist und diese Schlauchverbindung mittels des Verbindungsstrangs mit den anderen Schlauchverbindungen verbunden ist.

14. Das System gemäß eines beliebigen der vorhergehenden Ansprüche 8 bis 13, des weiteren **dadurch gekennzeichnet, dass**
der mikrobiell zu dekontaminierende Gegenstand ein Endoskop ist.

15. Das System gemäß Anspruch 14, des weiteren **dadurch gekennzeichnet, dass** die strangverbundene Einheit des weiteren Folgendes enthält:
eine Marke (54), durch die wenigstens (i) das Model des Endoskops oder (ii) die Familie von Endoskop-Modellen identifiziert wird, bei dem oder der die strangverbundene Einheit eingesetzt werden kann, wobei die Marke an dem Verbindungsstrang befestigt ist.

16. Das System gemäß Anspruch 15, des weiteren **dadurch gekennzeichnet, dass**
die Marke (54) Folgendes enthält:
eine schematisch Darstellung, die den korrekten Anschluss der an dem Verbindungsstrang angebrachten Schlauchverbindungen und Stöpsel an die Lumen-Einlassstutzen darstellt.

17. Das System gemäß eines beliebigen der vorhergehenden Ansprüche 12 bis 16, des weiteren **dadurch gekennzeichnet, dass**
die strangverbundene Einheit eine Vielzahl von Schlauchverbindungen und Stöpsel enthält, die alle durch den Verbindungsstrang zusammengehalten werden.

18. Das System gemäß Anspruch 17, des weiteren **dadurch gekennzeichnet, dass**
jede Schlauchverbindung und jeder Stöpsel einen Hinweis zu der jeweiligen Reihenfolge des Anschließens enthält und auch in dieser Reihenfolge an dem Verbindungsstrang angebracht ist.

19. Das System gemäß Anspruch 17, des weiteren **dadurch gekennzeichnet, dass**
der Gegenstand eine Vielzahl von Lumen-Einlassstutzen enthält;
jeder der Stöpsel und jede der Schlauchverbindungen so ausgelegt ist, dass er oder sie an einem entsprechenden Lumen-Einlassstutzen angeschlossen werden kann;
die Stöpsel und Schlauchverbindungen an dem Verbindungsstrang in entsprechender Reihenfolge und dabei entlang dem Verbindungsstrang so weit auseinander angebracht sind, dass jeder der Stöpsel und Schlauchverbindungen gerade den ihm oder ihr entsprechenden Lumen-Einlassstutzen an dem Gegenstand erreicht, und dass dabei zumindest einige der Stöpsel und Schlauchverbindungen nicht an irgendwelche Lumen des Gegenstandes heranreichen können, die ihnen nicht entsprechen.

20. Das System gemäß eines beliebigen der vorhergehenden Ansprüche 8 bis 19, des weiteren **dadurch gekennzeichnet, dass**
der erste Mikrobizid-Füllstutzen (26) Sprühdüsen enthält, die in zumindest zwei Gruppen betrieben werden, um dadurch das Bewegen zwischen den Fittings und Stöpseln und entsprechenden Lumen-Einlassstutzen zu erleichtern.

21. Das System gemäß eines beliebigen der vorhergehenden Ansprüche 8 bis 19, des weiteren **dadurch gekennzeichnet, dass**
das System einen Aufnahmetrog (312) zur Aufnahme des Gegenstands enthält, wobei der erste Mikrobizid-Füllstutzen (324) den Trog mit Flüssigkeit versorgt.

## Revendications

1. Procédé de désinfection ou de stérilisation d'un article (14) présentant des canaux intérieurs, ledit procédé comprenant les opérations consistant à placer l'article dans une chambre (10a, 10b), à raccorder à un orifice de sortie de fluide antimicrobien (28) une entrée de canal (51) de l'article donnant accès à ce canal, à mettre une surface extérieure de l'article en contact avec un fluide antimicrobien, et à faire couler le fluide antimicrobien dans le canal, ledit procédé comprenant les étapes consistant à :
établir le raccordement fluidique entre l'orifice de sortie de fluide antimicrobien et l'entrée de canal de l'article à l'aide d'un connecteur (56) présentant un embout (74) dont la configuration permet un raccordement lâche avec les surfaces contiguës à l'entrée de canal de sorte à ce qu'une première partie du fluide antimicrobien s'écoule par l'entrée de canal pour entrer dans le canal et qu'une seconde partie du fluide antimicrobien s'écoule entre l'entrée de canal et l'embout pour entrer dans la chambre, le raccordement étant suffisamment lâche pour que l'embout oscille, ce qui permet aux points de contact existant entre l'embout et les surfaces contiguës à l'entrée de canal de changer momentanément.

2. Procédé selon la revendication 1, **caractérisé en outre en ce que** :
l'article présente une pluralité de canaux et le connecteur (56) est raccordé au moyen d'une ligne d'attache (52) à au moins un de (i) un autre connecteur (56) et (ii) un obturateur (58), le procédé comprenant en outre :
le raccordement de chacun des connecteurs entre une sortie de fluide antimicrobien (28) et une entrée de canal (51), et
le raccordement de chacun des obturateurs (58) aux entrées des canaux (51).

3. Procédé selon la revendication 2, **caractérisé en outre en ce que** :
l'article devant subir la décontamination microbienne est un endoscope.

4. Procédé selon la revendication 3, **caractérisé en outre en ce que** :
une plaquette indicatrice (54) est attachée à l'un des éléments suivants : ligne d'attache (52), connecteur (56) et obturateur, laquelle plaquette indicatrice permet d'identifier au moins un de (i) un modèle d'endoscope ou (ii) une famille de modèles d'endoscope utilisé conjointement à l'ensemble de raccordement sur ligne d'attache.

5. Procédé selon la revendication 4, **caractérisé en outre en ce que** :
la plaquette indicatrice (54) comprend :
un schéma illustrant le raccordement convenable des connecteurs et des obturateurs sur ligne d'attache aux orifices de sortie du fluide et aux entrées des canaux.

6. Procédé selon l'une quelconque des revendications précédentes 2 à 5, **caractérisé en outre en ce que** :
chacun des connecteurs et obturateurs comprend des indications d'ordre de raccordement et est attaché à la ligne d'attache suivant cet ordre de raccordement, le procédé comprenant en outre :
le rattachement des connecteurs et obturateurs à leurs entrées de canal respectives dans l'ordre prescrit.

7. Procédé selon l'une quelconque des revendications précédentes 2 à 6, **caractérisé en outre en ce que** :
les obturateurs et connecteurs sont raccordés à la ligne d'attache suivant un ordre et un espacement appropriés le long de cette dernière de telle sorte que cette ligne d'attache permette à chacun des obturateurs et connecteurs d'atteindre précisément une entrée de canal respective existant dans l'article tout en n'atteignant pas une distance suffisante, pour au moins quelques uns des connecteurs et obturateurs, pour atteindre des canaux de l'article ne leur étant pas destinés.

8. Système de désinfection ou de stérilisation fluidique comprenant une chambre (10a, 10b) servant à la réception d'un article (14) comportant des canaux devant subir une décontamination microbienne, une pluralité d'orifices de sortie de fluide (26, 324) disposés dans la chambre et servant à apporter un fluide antimicrobien devant être mis en contact avec les surfaces extérieures de l'article comportant des canaux et au moins un orifice de sortie de fluide (28) à travers lequel peut être apporté le fluide antimicrobien destiné à un canal intérieur de l'article comportant des canaux, ledit système comprenant :
un connecteur (56) servant au raccordement fluidique dudit orifice de sortie de fluide avec une entrée de canal (51) de l'article afin d'apporter le fluide antimicrobien au canal intérieur, ledit connecteur comprenant :
un tube (71),
un premier embout (62, 64) raccordé à une extrémité du tube et permettant le raccordement audit orifice de sortie de fluide, et
un second embout (74) raccordé au tube et configuré pour être raccordé de manière lâche à l'entrée du canal de telle manière qu'il se forme un espace annulaire entre l'embout et l'entrée et que l'embout oscille, ce qui permet aux points de contact existant avec les surfaces contiguës à l'entrée de canal de changer momentanément de telle sorte que (i) une première partie du fluide antimicrobien s'écoule par l'entrée de canal pour entrer dans ce canal et que (ii) une seconde partie du fluide antimicrobien s'écoule entre l'entrée de canal et le second embout pour entrer dans la chambre.

9. Système selon la revendication 8, **caractérisé en outre en ce que** :
ledit orifice de sortie de fluide (28) comprend une vanne de fermeture qui présente un état ouvert et un état fermé non étanche permettant de fermer sensiblement mais pas totalement ledit orifice de sortie de fluide lorsqu'il n'est pas raccordé à un embout (62, 64).

10. Système selon l'une quelconque des revendications 8 à 9, **caractérisé en outre en ce que** :
un obturateur (58) est configuré pour être raccordé de manière lâche à une seconde entrée de canal (51) de telle manière qu'une partie du fluide antimicrobien s'écoulant dans le canal sorte par la seconde entrée de canal, entre l'obturateur et l'entrée.

11. Système selon la revendication 10, **caractérisé en outre en ce que** :
une ligne d'attache (52) permet le raccordement entre le connecteur et au moins l'obturateur (58) ; le connecteur (56) et l'obturateur restent attachés l'un à l'autre sous la forme d'un ensemble sur ligne d'attache (50).

12. Système selon l'une quelconque des revendications 8 à 10, **caractérisé en outre par** :
au moins un second connecteur (56) et
une ligne d'attache (52) permettant le raccordement entre le premier connecteur (56) et au moins un second connecteur (56) ; le premier et le second connecteur restent attachés l'un à l'autre sous la forme d'un ensemble sur ligne d'attache (50).

13. Système selon l'une quelconque des revendications 11 et 12, **caractérisé en outre en ce que** :
la chambre comprend en outre un orifice (36) raccordé à un détecteur de fuite (34), l'article possédant un orifice de test de fuite,
un ensemble tubulaire (56) présentant, à une extrémité, un embout (60) configuré pour être raccordé uniquement à un orifice (36) du détecteur de fuite et, à l'extrémité opposée, un autre embout (61) configuré pour être raccordé à un orifice de test de fuite de l'article, la ligne d'attache reliant l'ensemble tubulaire et le connecteur entre eux.

14. Système selon l'une quelconque des revendications 8 à 13, **caractérisé en outre en ce que** :
l'article devant subir la décontamination microbienne est un endoscope.

15. Système selon la revendication 14, **caractérisé en outre en ce que** l'ensemble de raccordement sur ligne d'attache comprend en outre :
une plaquette indicatrice (54) permettant d'identifier au moins un de (i) un modèle d'endoscope ou (ii) une famille de modèles d'endoscope devant être utilisé conjointement à l'ensemble, ladite plaquette indicatrice devant être reliée à la ligne d'attache.

16. Système selon la revendication 15, **caractérisé en outre en ce que** :
la plaquette indicatrice (54) comprend en outre :
un schéma illustrant le raccordement convenable des connecteurs et des obturateurs sur ligne d'attache aux entrées des canaux de l'endoscope.

17. Système selon l'une quelconque des revendications 12 à 16, **caractérisé en outre en ce que** :
l'ensemble sur ligne d'attache comprend une pluralité de connecteurs et une pluralité d'obturateurs qui sont tous reliés par la ligne d'attache.

18. Système selon la revendication 17, **caractérisé en outre en ce que** :
chacun des connecteurs et obturateurs comprend des indications d'ordre de raccordement et est attaché à la ligne d'attache suivant cet ordre de raccordement.

19. Système selon la revendication 17, **caractérisé en outre en ce que** :
l'article comprend une pluralité d'entrées de canal,
chacun des obturateurs et connecteurs est configuré pour être connecté avec une entrée de canal respective,
les obturateurs et connecteurs sont raccordés au moyen de la ligne d'attache suivant un ordre et un espacement appropriés le long de la ligne d'attache de telle sorte que cette ligne d'attache permette à chacun des obturateurs et connecteurs d'atteindre précisément une entrée de canal respective existant dans l'article tout en n'atteignant pas une distance suffisante, pour au moins quelques uns des connecteurs et obturateurs, pour atteindre des entrées de canal de l'article ne leur étant pas destinées.

20. Système selon l'une quelconque des revendications précédentes 8 à 19, **caractérisé en outre en ce que** :
le premier orifice de sortie de fluide (26) comprend des buses de pulvérisation qui fonctionnent en au moins deux groupes, ce qui facilite le mouvement entre les embouts, les obturateurs et les entrées de canal respectives.

21. Système selon l'une quelconque des revendications précédentes 8 à 19, **caractérisé en outre en ce que** :
un plateau de réception (312) reçoit l'article et le premier orifice de sortie de fluide (324) fournit le fluide au plateau.
